# EUROPEAN PATENT APPLICATION

(11) **EP 4 268 899 A2**
(43) Date of publication of application: **01.11.2023**
(21) Application number: 23184063.8
(22) Date of filing: 31.10.2019
(51) Int. Cl.: A61P 35/00

(54) **FERRITIN NANOPARTICLES COMPRISING A CHEMOTHERAPEUTIC AGENT**

(30) Priority: 31.10.2018 IT 201800009959
(62) Divisional of application: 19808536.7
(71) Applicant: Coleman Srl, 20861 Brugherio (MB) (IT)
(72) Inventor: MAZZUCCHELLI, Serena, 21012 Cassano Magnago (IT); CORSI, Fabio, 20090 Buccinasco (IT); TRUFFI, Marta, 21013 Gallarate (IT); PROSPERI, Davide, 20158 Milano (IT); COLOMBO, Miriam, 20142 Milano (IT); BELLINI, Michela, 24047 Treviglio (IT)
(74) Representative: Zaccaro, Elisabetta

(57) **Abstract**

The present invention concerns the field of cancer therapy, and in particular to the use of nanoparticles for the preservation of T cells.

In particular, the present invention relates to the use of nanoparticles for the treatment of recurrent cancer and for use as a cancer vaccine.

## Description

### FIELD OF THE INVENTION

The present invention concerns the field of cancer therapy, and in particular to the use of nanoparticles for anticancer purposes with preservation of T cells.

In particular, the present invention relates to the use of nanoparticles for the treatment of recurrent cancer and for use as a cancer vaccine.

### STATE OF THE ART

Effective clearance of the same pathogen upon reinfection can be achieved as a result of the ability of the immune system to remember its response to the same pathogens. The immune system has an innate memory, the immunological memory, which is at the basis of this ability.

Naive T cells, produced by the thymus, are the immune cells which start the memory response to a pathogen. Each Naive T cell has a unique T-cell receptor which recognizes a specific part of the pathogen.

Only those naive T cells that recognize a pathogen via their T-cell receptors lose their naivety and differentiate into effector cells, which can kill infected cells or give help to other immune cells. Following pathogen clearance, most of the effector cells will die while a small pool of T cells develops into memory T cells, responsible for the quicker and stronger immune response upon a subsequent encounter of the pathogen. Interestingly, not only pathogens are recognized by the immune system, but also cancer cells due to their expression of several "non-self' antigens. Cancer treatments, such as chemotherapy, can weaken the immune system because they can cause a drop in the number of white blood cells made in the bone marrow and reduce the number of neutrophils in the body, making it harder to fight infections.

Furthermore, chemotherapy acts on T cells, reducing their number and activity, impairing their ability to differentiate into fighter T cells, which would be the defense against tumor metastasis.

Recurrent cancer is a tumor that comes back after initial treatment. Although the initial treatment is aimed at eliminating all cancer cells, a few may have evaded treatment and survived. These undetected cancer cells might proliferate, becoming recurrent cancer, which may occur months or years after initial treatment. The cancer may come back in the same place as the original cancer (local recurrence), or it may spread to distant organs and tissues (distant recurrence).

The presence of an unaltered population of T cells could provide active surveillance against recurring cancer and metastasis. It is therefore essential to avoid T cell loss during the first cancer treatment in order to preserve this cell population.

The need and importance is increasingly felt for an effective way of conveying the chemotherapeutic drug to the tumor cells without acting on and decreasing the T cell functionality.

### SUMMARY OF THE INVENTION

As will be further described in the detailed description of the invention, the problem underlying the present invention is the targeted treatment of cancer, thereby avoiding cytotoxicity of anticancer treatments on patient's T cells population.

In order to achieve this, the present invention concerns a ferritin nanoparticle comprising a chemotherapeutic agent, for use in the preservation of T cells in a cancer patient.

The present invention further relates to a ferritin nanoparticle comprising a chemotherapeutic agent, for use in the treatment of recurrent cancer.

In a further embodiment, the invention describes the use of a ferritin nanoparticle comprising a chemotherapeutic agent, as a cancer vaccine.

### BRIEF DESCRIPTION OF THE DRAWINGS

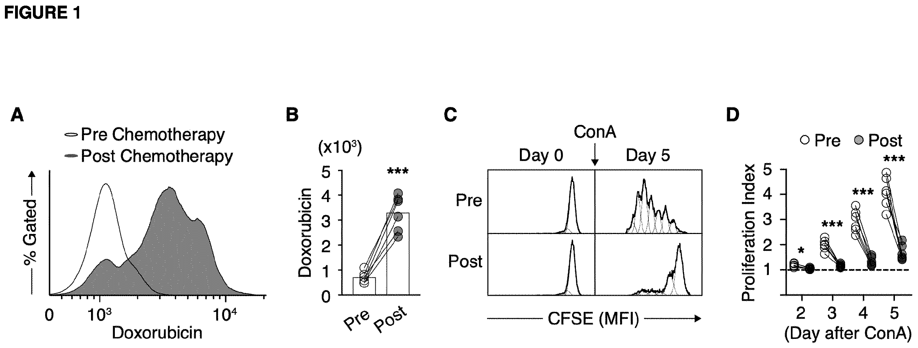

The characteristics and advantages of the present invention will be apparent from the detailed description reported below, from the Examples given for illustrative and nonlimiting purposes, and from the annexed Figures 1-3, wherein:
**Figure 1****:** DOX effects on proliferation potential of PBMC from patients under neoadjuvant chemotherapy.
   A. Flow cytometry of DOX internalization profile in PBMC isolated from a representative patient before and after DOX parenteral infusion.
   B. Quantification of Mean Fluorescence Intensity (MFI) in PBMC isolated from patients. The profiles represent the values of DOX fluorescence in each patient before and after chemotherapy. The histogram reports the mean DOX fluorescence value between all the samples analyzed. N=3, Unpaired t-test *p<0.05, **p<0.01, ***p<0.001.
   C. Representative image of proliferation analysis of PBMC isolated from BC patients before and after 1 cycle of neoadjuvant chemotherapy with DOX using CFSE assay.
   D. Quantification of proliferative index at different days of culture after Concanavalin A stimulation (ConA). The proliferation index is the ratio between proliferating cells and progenitor cells. The absence of proliferation is represented by a proliferative index of 1. N=3, Unpaired t-test *p<0.05, **p<0.01, ***p<0.001.
**Figure 2****:** Characterization of lymphocytes subpopulations that internalize DOX. Flow cytometry immunophenotyping of T- Lymphocytes CD8+ e CD4+ to identify populations that internalize DOX (high, int, low). CM: Central Memory, EM: Effector Memory.
**Figure 3****:** Internalization of PBMC formulated in H-ferritin (HFn) nanocages.
   A. Flow cytometry quantification of Transferrin Receptor 1 (TfR1) expression in different populations of T-lymphocytes isolated form PBMC of healthy donors. Dashed line represent the MFI of the control used to set the basal level of fluorescence (mouse lgG1).
   B. Kinetics of interaction of DOX free or nanoformulated in HFn (HFn-DOX) in human PBMC.
   C. Intracellular localization of DOX e HFn-DOX by confocal microscopy.
   D. Analysis of lymphocyte populations that internalize DOX before and after incubation with DOX or HFn-DOX.
   E. Proliferation assay using CFSE in PBMC incubated for 24 h with DOX or HFn-DOX (1µM) and stimulated with ConA for 5 days. PI: Proliferation index.

### DETAILED DESCRIPTION OF THE INVENTION

The present invention concerns the field of cancer treatment and in particular relates to solving the problem of avoiding the impairment of the immune system following the chemotherapeutic treatment. It has been observed that cancer patients have a dramatic decrease in the number and the functionality of T cells following chemotherapy.

Object of the present invention is thus a ferritin nanoparticle comprising a chemotherapeutic agent, for use in the preservation of T cells in a cancer patient.

Ferritin is a ubiquitous protein found in eubacteria, archea, plants and animals including humans, but not in yeast. It exists in extracellular and intracellular compartments, such as the cytosol, nucleus and mitochondria and its main roles are iron storage and homeostasis. Therefore, it is one of the most highly conserved molecules.

Ferritin is a large protein of 450 kDa composed of 24 subunits that self-assemble into a spherical cage-like structure with inner and outer dimensions of 8 and 12 nm, respectively. Eukaryotes have two ferritin genes encoding the heavy (H; 21 kDa) and the light (L; 19 kDa) chains. The H-chain is responsible for the oxidation of Fe(ll) to Fe(lll) and includes the ferroxidase catalytic site, while the L-chain plays a role in iron nucleation. H and L chains co-assemble into a 24-mer heteropolymer, where the H-chain to L-chain ratio varies according to a tissue specific distribution.

Ferritin protects cells from the damage caused by the Fenton reaction during oxidative stress, by sequestering Fe2+, a source of toxic reactive oxygen species, and converting it into harmless Fe3+, which is stored as ferrihydrite crystals.

The ferritin protein cage can accommodate up to 4500 iron atoms. This protective action is crucial in the nucleus, where the DNA has to be particularly preserved from iron-induced oxidative damage.

Extracellular ferritin interacts with cells through the receptor of transferrin 1 (TfR1), which specifically binds H-ferritin, while L-ferritin shows low interaction. After binding on the cell surface, the H-ferritin-TfR1 complex is internalized, and can be found in early and recycling endosomes, demonstrating that TfR1 coordinates the processing and the use of iron by binding both Transferrin and H-ferritin. H-ferritin is also able to interact with the T-cell Immunoglobulin and Mucin domain-2 (TIM-2), which is overexpressed in oligodendrocytes and B-cells, and is internalized in endosomes upon binding. H-ferritin, which naturally interacts with the TfR1, are the most extensively studied and they are used to design naturally targeted nanoparticles for nanomedicine applications by exploiting the TfR1 overexpression in many types of tumor cells.

Recombinant ferritin provides a central cavity, which can be efficiently loaded with transition metals, drugs, fluorescent molecules or contrast agents. Since it has a uniform cage, ferritin allows the precise control of the amount of encapsulated molecules, which is a critical feature in defining drug dosage. Moreover, the protein shell of ferritins can be easily modified either chemically or genetically to introduce different functionalities. All of these attributes highlight ferritin and its derivatives as powerful systems with potential application in nanomedicine.

By the term "nanoparticle" or "HFn nanocage" as used herein it is intended to comprise a cage-like structure, a shell or a composition comprising ferritin and a chemotherapeutic agent. Said chemotherapeutic agent is preferably enclosed within the nanocage. The ferritin nanoparticle comprising a chemotherapeutic agent can also be indicated as a ferritin nanocage with an encapsulated chemotherapeutic agent.

The ferritin quaternary structure has eight hydrophilic channels that seem to mediate iron transit in and out of the protein cage. There are also six hydrophobic channels, which do not seem to be involved in iron exchange although they could mediate the transit of protons. In spite of the structural rigidity of ferritins in the physiological environment, the protein cages can be reversibly disassembled when the pH becomes extremely acidic (pH 2-3) or basic (pH 10-12). When the pH returns to neutrality ferritin monomers are able to self-assemble in a shape memory fashion. Moreover, the ferritin cage is resistant to denaturants, including heating to high temperatures (>80 °C).

Extracellular ferritin interacts with cells through the receptor of transferrin 1 (TfR1), which specifically binds H-ferritin, while L-ferritin shows low interaction. After binding on the cell surface, the H-ferritin-TfR1 complex is internalized, and can be found in early and recycling endosomes, demonstrating that TfR1 coordinates the processing and the use of iron by binding both Transferrin and H-ferritin. H-ferritin is also able to interact with the T-cell Immunoglobulin and Mucin domain-2 (TIM-2), which is overexpressed in oligodendrocytes and B-cells, and is internalized in endosomes upon binding. H-ferritin, which naturally recognizes TfR1, are the most extensively studied and they are used to design naturally targeted nanoparticles for nanomedicine applications by exploiting the TfR1 overexpression in many types of tumor cells.

In a further aspect, the present invention relates to the use of the ferritin nanoparticle comprising a chemotherapeutic agent in the preservation of the T cells, of the present invention, wherein said cancer patient is a patient suffering from breast cancer, bladder, lung, stomach, ovarian cancer; Hodgkin's lymphoma, non-Hodgkin's lymphoma, certain types of leukemia, including acute lymphoblastic leukemia (ALL) and acute myeloid leukemia (AML, ANLL). It is used with other drugs in the treatment of certain types of thyroid cancer and certain types of soft tissue or bone sarcomas, neuroblastoma and Wilms' tumor.

In a preferred aspect, the ferritin nanoparticle comprising the chemotherapeutic agent is for use in the preservation of the T cells of a patient suffering from breast cancer.

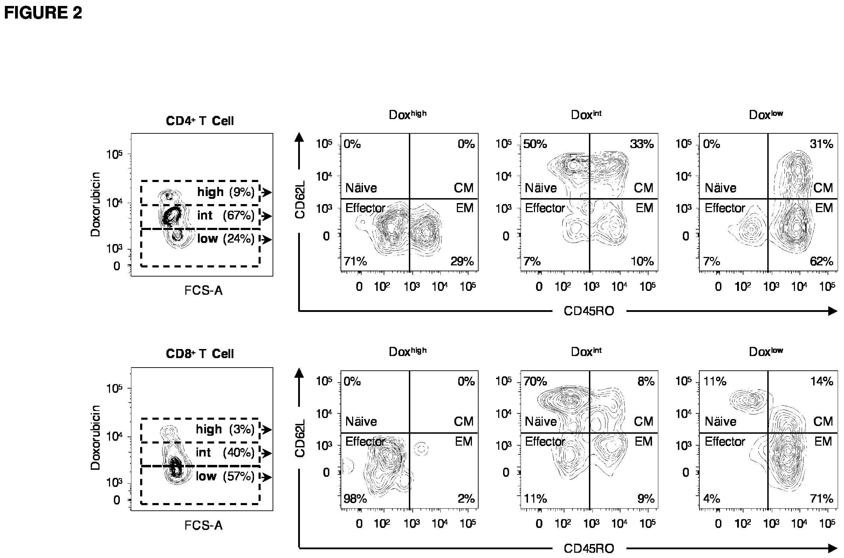

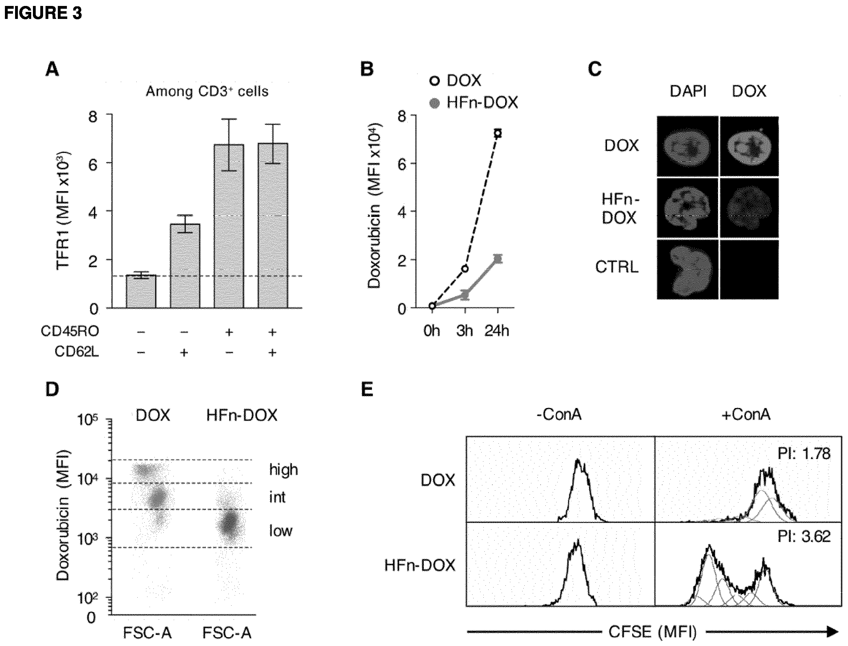

In a further preferred aspect, the chemotherapeutic agent of the ferritin nanoparticle is chosen from the group consisting of doxorubicin (DOX), Platinum derivatives, taxane, PARP-1 inhibitors, mTOR inhibitors, PI3K inhibitors, benzothiazoles, curcuminoids, desferoxamine B. We explored the phenotypic and functional effects of DOX on matched-paired lymphocytes coming from breast cancer (BC) patients before and after neoadjuvant chemotherapy. We found that patient-derived PBMC, isolated after chemotherapy, promptly internalized DOX and displayed a dramatic proliferative impairment in response to mitogenic stimulations compared to the match-paired PBMC isolated before the treatment (Figure 1). Interestingly, FC characterization showed that distinct lymphocytes sub-populations internalized DOX in a different magnitude, where naive T-cells (CD3+CD8+/CD4+CD45RO-) displayed the highest DOX uptake (Figure 2). Since pivotal to every adaptive immune response is the activation and massive proliferation of T cells from their resting state, we have exploited a DOX formulation in ferritin nanocages (HFn-DOX) - whose uptake is specifically mediated by transferrin receptor 1 (TfR-1) - in order to circumvent drug internalization in those immune cells. The fact that T cells, and in particular naive T cell,s express low levels of TfR-1, allowed us to show the capability of HFn-DOX to protect lymphocytes from DOX exposure meanwhile preserving their competence to generate an immune response (Figure 3). These findings were corroborated in vivo in a murine model of breast cancer in which weekly intravenous injections of HFn-DOX controlled tumor growth and, at the same time, avoided off-targeting in secondary lymphoid organs. As a whole, our data indicate that preventing chemotherapy-related toxicity on T cells through HFn-DOX could allow the host adaptive immune system to fully benefit from the DOX-induced immunogenic cell death to vaccinate against cancer.

A further object of the present invention relates to the use of ferritin nanoparticles comprising a chemotherapeutic agent, as a cancer vaccine.

Without being bound to any theory, preliminary findings suggest that:
a) in HFn-DOX treated patients, these neoantigens could be phagocytosed and then exposed by an Antigen-Presenting Cell (APC). The tumor neoantigen exposed on APC surface is used to select the appropriate clone of T- cells able to recognize that antigen, and capable of triggering the anti-cancer immune response followed by this "new vaccination" approach.
b) In DOX treated patients, T-cells are not yet functional and prone to trigger the clonal expansion necessary to mediate the anti-cancer immune response, despite these neoantigens have been produced, phagocytosed by APC and then exposed on their surface. In a preferred aspect, said cancer vaccine is against breast cancer and said chemotherapeutic is doxorubicin.

Despite the natural targeting of ferritin towards cancer cells, the surface of ferritin nanocages has been modified by inserting a number of target motifs, such as antibodies, peptides and antibody fragments, in order to drive selective recognition of specific cell subtypes. Moreover, their high stability, biocompatibility, ability to disassemble and reassemble in a shape memory fashion and disposition for surface modification make ferritin nanoparticles an ideal platform for drug delivery.

Indeed, in physiological conditions ferritin has a stable 24-mer cage architecture, while in highly acidic and basic conditions its quaternary structure disassembles, and then reassembles when the pH of the solution is brought to neutrality allowing the encapsulation of molecules in solution within ferritin cavities, simply by disassembling and reassembling the 24-mer architecture.

Drugs with a natural tendency to bind metals, such as cisplatin and desferoxamine B, have been easily entrapped in the ferritin shell. However, most of the currently used chemotherapies are not based on metals such as cisplatin, and the incorporation of non-metal-containing drugs within ferritin is complicated by their limited interaction with the protein cage.

Doxorubicin (DOX) encapsulation in ferritin nanocages represents the most extensively investigated system for delivery of anticancer drugs. DOX pre-complexed with Cu(ll) has been loaded inside ferritin nanocages and evaluated *in vitro* and *in vivo* in U87MG glioblastoma tumor models.

It has been now surprisingly shown that the ferritin nanoparticle comprising a chemotherapeutic agent (ferritin nanocages with encapsulated chemotherapeutic agent), such as doxorubicin, allow for the preservation of the immune system cells, and in particular the naive T cells in a cancer patient.

Eighty percent of DOX was gradually released from RGD-functionalized apoferritin within 10 hours of incubation at 37 °C in phosphate buffered saline (PBS), while the copper remained bound to the internal surfaces of the nanocages. This targeted nanoformulation has increased drug tumor uptake and accumulation, tumor growth inhibition, circulation half-life, and has reduced the cardiotoxicity induced by free DOX. Other groups have set up DOX loading strategies that do not involve complexes with transition metals. In these cases, good encapsulation efficiency has also been obtained, and excellent results in terms of tumor growth inhibition and reduced toxicity have been reported, both *in vitro* and *in vivo. In vivo* results clearly demonstrated that ferritin nanocages improve DOX bioavailability, tumor accumulation and clearance, and suggested that ferritin-mediated active targeting provides a major contribution. The mechanisms and kinetics of drug release from ferritin shell has not been completely elucidated, although reported data with DOX suggest that encapsulation is stable in serum and that a pH-triggered release takes place in vitro.

In still further a preferred aspect, in the use of the ferritin nanoparticle comprising the chemotherapeutic agent for the preservation of the naive T cells, said cancer patient suffering from a primary cancer or from a recurrent cancer.

In particular, it has been surprisingly seen that the incubation of PBMC from healthy donors with the nanoparticle comprising the chemotherapeutic agent allows for the preservation of the PBMC. Indeed, the MFI due to DOX uptake is lower in PBMC treated with the nanoparticle comprising the chemotherapeutic than in PBMC treated with the free drug (figure 3B). This different uptake is coupled with a different accumulation in the nuclear compartment, which is the DOX site of action figure 3C). Moreover, the treatment with ferritin nanoparticle comprising the chemotherapeutic agent allow to identify only one population with low DOX uptake (figure 3D), probably as a consequence of the moderate expression of the TfR1 in these cells (Figure 3A). Therefore, in samples treated with ferritin nanoparticle comprising the chemotherapeutic agent the proliferation index remains high (figure 3E).

Furthermore, it has been surprisingly found that the treatment of the patient with the nanoparticle comprising the chemotherapeutic agent of the present invention does not induce harmful effects such as hemolysis in erythrocytes and is not toxic on liver, kidneys, spleen, brain, gut and lung.

A severe drawback of doxorubicin chemotherapy is in fact cardiotoxicity. The nanoparticles according to the present invention have been surprisingly seen to avoid this problem since DOX treatment has been demonstrate to cause a severe and irreversible cardiotoxicity of type I. The cardiosafety observed in samples treated with ferritin nanoparticle comprising the chemotherapeutic agent allows to hypothesize the combination with other very effective but cardiotoxic drugs such as the monoclonal antibody Trastuzumab.

In a further embodiment, the present invention relates to a ferritin nanoparticle comprising a chemotherapeutic agent, for use in the treatment of the recurrence of cancer.

In a preferred aspect the Ferritin nanoparticle comprising a chemotherapeutic agent, is for use in the treatment of the recurrence of cancer.

In a still preferred aspect the Ferritin nanoparticle comprising a chemotherapeutic agent, is for use in the treatment of recurrent breast cancer bladder, lung, stomach, ovarian cancer; Hodgkin's lymphoma, non-Hodgkin's lymphoma, certain types of leukemia, including acute lymphoblastic leukemia (ALL) and acute myeloid leukemia (AML, ANLL).

It is used with other drugs in the treatment of certain types of thyroid cancer and certain types of soft tissue or bone sarcomas, neuroblastoma and Wilms' tumor.

In a still more preferred aspect, the ferritin nanoparticle for use in the treatment of recurrent cancer comprises a chemotherapeutic agent, is doxorubicin, Platinum derivatives, taxane, PARP-1 inhibitors. mTOR inhibitors of mTOR, PI3K inhibitors, benzothiazoles, curcuminoids, and desferoxamine B.

Also disclosed herein is a method for the treatment of a subject suffering from cancer, comprising the steps of:
- administering a ferritin nanoparticle comprising a chemotherapeutic agent to said subject,
wherein said treatment is for the preservation of the T cells of said subject.

In a preferred aspect, said method relates to the preservation of the T cells of said subject suffering from breast cancer and undergoing the treatment with a chemotherapeutic agent, comprising the steps of:
- administering a ferritin nanoparticle comprising a chemotherapeutic agent to said subject.

In a still preferred aspect, said method relates to the preservation of the T cells of said subject suffering from breast cancer and undergoing the treatment with doxorubicin, comprising the steps of:
- administering a ferritin nanoparticle comprising doxorubicin to said subject.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention.

### Example 1.

### HFn-DOX production: HFn design, synthesis, loading and characterization.

A ferritin nanoparticle also indicated herein as "HFn nanocage" according to the present invention has been produced by DNA recombinant technology in E. coli, after the design of an appropriate expression vector. The reverse complement of the cDNA encoding for the heavy chain of human ferritin was modified by inserting the restriction sites for Notl and Ndel (in 5' and 3' respectively) and synthesized by Eurofins MWG Operon. The resulting DNA fragment was subcloned into the pET30b(+) vector between Notl and Ndel restriction sites to express the HFn under the control of the T7 promoter. The resulting plasmid pET30b(+)/HFn was used to transform by heat-shock the E. coli expression strain BL21(DE3) and transformed clones were selected for kanamycin resistance.

HFn production procedure was set up in 1 l flask and then scaled up in a 1L bioreactor preserving mostly the same procedure of protein production and purification. Transformed cells were grown at 37 °C in LB/kanamicin medium under shaking at 220rpm until Lag phase (OD600nm = 0.6). Then, protein production was induced with 0.5 mM IPTG for 2 h 30 min and cells were collected by centrifugation at 4000 ×g for 15 minutes and washed with PBS buffer. We obtained 4-4.5 g/l culture by the standard procedure, while using the bioreactor we were able to obtain a pellet of 20-22 g/L culture. Cells were resuspended in lysis buffer supplemented with Lysozyme and DNAsel, sonicated to disrupt cell membrane and centrifuged to collect the crude extract. The crude extract was subjected to heat-treatment for 15 min at 70 °C, which denatures almost all proteins except for HFn. The supernatant was then loaded onto DEAE Sepharose anion exchange resin, preequilibrated with 20mM KMES, pH 6.0 and HFn was eluted with a stepwise Sodium chloride gradient, from 70 mM to 420 mM, in 20mM KMES, pH 6.0. The yield of the process is about 56 mg/L culture.

The purification procedure is mostly the same with the pellet produced in 1 L bioreactor. In this case the pellet was divided into three aliquots of about 7 g each. Moreover, the sonication process was replaced by French press and the bed volume for chromatography was increased to 10 mL.

This process results in a three fold increase in HFn production after scale-up with 1 L bioreactor system. To increase HFn purity we introduced an additional step consisting of a FPLC gel filtration using Superose 6 Increase 10/300 GL column. Protein gel electrophoresis performed in native conditions and transmission electron microscopy images revealed that the structural integrity of the HFn nanocage is retained after purification. DLS profiles evidenced a uniform monodisperse population with a mean Hydrodynamic size of 11.98. HFn is negatively charged with a ζ-potential of -24.8 ± 0.8 mV. Mycoplasma assay and bacterial culture reveal that the HFn product is free of contamination. Moreover, the TNF alpha activation assay revealed that FPLC purification step is necessary to avoid TNFalpha-activation.

HFn loading with doxorubicin was performed using the pH-dependent disassembling procedure. A solution of 200 µM doxorubicin hydrochloride (8 µmol) was added to a HFn solution (20 mg, 0.5 mg mL-1 in 0.15 M NaCl) and adjusted to pH 2.0 by 0.1 M of chloridric acid (HCl). Then, the pH was maintained for about 15 min and when the dissociation of HFn was completed, the pH value was increased up to 7.5 using 0.1 M Sodium idroxide (NaOH).

The resulting solution was stirred at room temperature for 2 h. In order to remove the excess of doxorubicin (DOX) and the adsorbed molecules, the solution was loaded onto a ZEBA spin desalting column (Thermofisher). This procedure is reproducible and allows to recover about 25% of incubated HFn. The recovery of DOX is about 27%. The mixture of HFn-DOX (ferritin nanoparticle comprising doxorubicin) could be concentrated up to a concentration of 400 µg/mL DOX. The nanodrug can be stored at 4 °C for short storage, or at -20 °C for long storage. Native protein gel electrophoresis and TEM images evidenced that the structural integrity of the HFn-DOX nanocage is retained after loading. DLS profiles evidenced a uniform monodisperse population with a mean Hydrodynamic size of 12.44± 1.1 nm. HFn is negatively charged with a ζ-potential of -25t 0.9 mV.

This product is stable at 4°C up to 1 month, as demonstrated by DLS. When stored at - 20°C the stability is longer. We have evaluated it over 8 months.

### Example 2.

### HFn-DOX in vitro and in vivo antitumor activity

Regarding *in vitro* activity, HFn interaction with cancer cells was assessed by flow cytometry after incubation of FITC-labelled HFn with a panel of murine and human cell lines of cancer, mainly breast cancer, for 2 h at 4 °C. HUVEC cells were selected as healthy cells with high TfR1 expression. Competition assay performed using unlabelled HFn as competitor revealed that the interaction between HFn and cell lines is specific. Confocal microscopy evidenced that HFns are quickly internalized and colocalize with the transferrin receptor 1.

The HFn-DOX nanodrug is more effective in reducing cancer cell viability in comparison to free DOX, as reported in viability assays performed with 4T1 and HeLa cells.

Then, we have assessed the *in vivo* activity of HFn-DOX nanodrug. We have used as proof of concept an ortotopic allograft breast cancer model obtained by injection of 4T1 cells in the mammary fat pad of 6-8 weeks old BALB/c female mice. This model is characterized by high level of proliferation, migration and invasiveness, DOX-inducible expression of MDR-1 (or P-glycoprotein) and stable luciferase expression. First, we have evaluated tumor targeting and biodistribution of the void HFn. HFn labelled with Alexa fluor 660 were injected into the tail vein of tumor bearing mice. At different time points, the fluorescent signal of HFn was recorded *in vivo* and ex *vivo* on tumor samples revealing that the HFn reaches the tumor 1h after injection. There is then a quick disappearance of the fluorescent signal. Confocal images on tumor cryosections revealed that the HFn goes deep into the tumor tissue, as demonstrated by intracellular localization of the green signal.

The biodistribution study evidenced a massive accumulation in the liver, although this signal completely disappears 48h after injection. Lung, brain and heart don't seem to be reached by HFn, while there is a little localization in kidneys and spleen within the first two hours post injection.

The presence of a fluorescence signal in the kidneys, coupled with the one in bladder and urine strongly suggests renal excretion of HFns. About HFn-DOX, we assessed drug bioavailability, by measuring by mass spectrometry the amount of Doxorubicin and doxorubicinol in plasma at different time points after drug administration, in comparison to free DOX and CAELYX, which is a DOX liposomal formulation.

Liposomal DOX improves DOX bioavailability, while the nanoformulation in HFn is similar to free DOX. However, the liposomal DOX exhibits also a massive increase in the amount of doxorubicinol, which is the most toxic derivative of DOX. Tumor accumulation of HFn-DOX is similar to that observed for free DOX, suggesting that there isn't an improvement in tumor drug delivery.

The nanoformulation in HFn nanocages improves DOX biodistribution in comparison to free DOX, reducing the amount of DOX and DOXol accumulated in non-target organs, and excreted in urine. In our setting HFn-DOX, free DOX, liposomal DOX or placebo were administered to tumor bearing mice at day 5, 9, 13 and 17 after cell implant in a dosage of 1.24 mg/kg (about 20 fold less of the therapeutic dosage of DOX in mice models).

Both the nanoformulations significantly reduced tumor progression, by affecting the number of apoptotic cells in the tumor tissue, and also tumor angiogenesis as demonstrated by CD31 immunohistochemistry on tumor samples dissected at day 21. HFn-DOX doesn't stimulate MDR-1 overexpression, and hence chemoresistance. For assessing the toxicity, we performed the hemolysis assay measuring the absorbance of the Heme group. We verified that the nanoformulation in HFn nanocages is not able to induce hemolysis in erythrocytes. The histopathological evaluation performed on liver, kidneys, spleen, brain, gut and lung dissected at day 21 from mice treated with HFn-DOX, DOX, liposomal DOX and placebo confirmed the general safety of HFn-DOX in these organs.

The evaluation of liver functionality assessed by AST and ALT assay confirmed that HFn-DOX is safe in the tested conditions. The creatinine/urea ratio was used to test kidney functionality, suggesting that it is unaffected by the treatment with HFn-DOX. Finally, we have focused our analysis on cardiotoxicity, which is the main and most severe drawback of doxorubicin chemotherapy. Our analysis evidenced that HFn-DOX doesn't induce the increase in cardiomyocyte cross-section area, which is the first signal of cardiac damage observed with DOX and liposomal DOX. Analyzing in detail the morphology of the heart tissue, we observed an increase in mitochondria number in DOX and liposomal DOX treated samples, in comparison to placebo. This was not registered in HFn-DOX treated samples. Moreover, the increase in mitochondria number was coupled with alteration in mitochondria area and cristae morphology, which were not registered in HFn-DOX treated samples. DOX and Liposomal DOX treatment induced alterations in mitochondrial membrane potential and in the amount of reduced glutathione, which are signals of abnormal mitochondrial functionality. Instead, HFn-DOX displayed a behavior similar to placebo.

Since HFn-DOX would allow to treat patients enhancing DOX anticancer activity while minimizing cardiotoxicity. Our idea is that kind of nanodrug could be innovative in the clinical approach on HER2 positive breast cancer, since in the treatment of this cancer subtype there are two highly effective drugs, Trastuzumab and doxorubicin, that can not be administered in combination due to their severe cardiotoxicity, as demonstrated by the NOAH trials. Indeed, blocking HER2 signaling with Trastuzumab prevents activation of the signaling pathways that mediate cardiomyocyte growth and cardiac repair mechanisms, resulting in vulnerability to cardiac stress. Anthracycline exposure exerts direct cytotoxicity against cardiac myocytes, resulting in severe cardiotoxic effects of these agents. Therefore, inhibition of HER2 dimerization in association with anthracycline therapy could exacerbate anthracycline-induced cardiac damage. Nanomedicine answers to this clinical issue with HFn-DOX represents the answer of nanomedicine to this clinical issue.

HER2+ BC bearing mice have been treated 5 times twice a week with placebo, HFn-DOX (1 mg/Kg, i.v.), TZ (5 mg/Kg, i.p.) and with the combination of them. Main end-point were cardiotoxicity and anticancer efficacy. Although single treatments with HFn-DOX or TZ display a good capability to reduce tumor progression, their combination improves antitumor potential, affecting tumor size and angiogenesis. Since the main TZ activity is the induction of the Antibody-Dependent Cell mediated Cytotoxicity, we have assessed the effect of HFn-DOX on Tumor Infiltrating Lymphocytes (TIL), revealing that both TILs enumeration and TIL activity is unaffected by HFn-DOX. On the other hand, HFn-DOX increases the induction of apoptosis, suggesting that the reduction of the tumor size observed in mice treated with the combination of TZ and HFn-DOX is attributable to the coupling of these activity. Mitochondrial morphology has been checked for cardiotoxicity.

A pathological increase in mitochondria area coupled with cristae depletion has been evidenced only in mice treated with TZ alone, confirming the overall safety of the HFn-DOX formulation. Interestingly, mice treated with the TZ and HFn-DOX did not display evidences of cardiac suffering. TZ quantification in tumor and heart revealed that the combination with HFn-DOX couples the increased TZ accumulation and penetration in tumor with TZ reduction in heart, resulting in the lack of cardiotoxicity. Our results suggest that a combined therapy with HFn-DOX and TZ allows an enhanced anticancer activity and reduced cardiotoxicity, with potential translational implications on the treatment of HER2+ BC.

### Example 3.

### Innovation: DOX formulation able to preserve immune competence of T-cells

Breast Cancer (BC) is the most frequently diagnosed malignancy and the second leading cause of cancer-related death among women. Anthracyclines such as doxorubicin (DOX) are considered a mainstay in chemotherapy for BC treatment due to their excellent cytotoxic activity. Indeed, DOX displays a direct cytotoxicity in BC cells by directly interfering with DNA replication and thus it is very effective against highly proliferating cancers. Besides its direct killing activity, DOX acts enhancing tumor immunogenicity: DOX induces the immunological cancer cell death (ICCD) that facilitates antigen presentation by dendritic cells (DC). Indeed, cancer cells are recognized as non-self by the immune system, since they express particular Tumor Asscociated Antigens and/or Neoantigens. The first ones are recognized as non-self since they are expressed in aberrant manner in tumors, while the Neoantigens are proteins which carry tumor-specific mutations, as a result of the high genomic instability of cancer, which leads to progressive accumulation of mutated genes. Upon cancer cell death occurring during treatment with DOX-based chemotherapy, several cancer neo-antigens become immediately available and activate DCs. DCs in turn stimulate rare naive cancer-specific T-cells maturation and clonal expansion, in order to induce an adaptive-antitumor immune response. Antitumor immunity could be critical to obtain complete remission and to generate a successful immunological memory (IM) that can surveil for relapses and/or metastases. This mode of antitumor immunity development, on which it is based the concept of "in situ vaccine", is intensely pursued by both researchers and clinicians, because: 1) neoantigens released during immunogenic cancer cells death resemble all the antigenic repertoire of BC, thus avoiding possible tumor escape from immune response; 2) this type of "cancer vaccination" is personalized and it does not need to identify universal cancer antigens; 3) it is a relatively simple exploitation of anticancer immunity strategy; 4) the most aggressive BC, as TNBC and HER2-positive are highly immunogenic cancers, therefore this strategy could be particularly effective especially in neoadjuvant setting.

However, some major concerns today limit the application of this immune-strategy for DOX- treated BC. First, there is a paradox regards on immune implications of DOX-treatment: DOX allows and stimulates ICCD that is essential to trigger this antitumor immune response, but at the same time it is restricted by DOX itself. Indeed, T-cells clonal expansion is one of the most proliferative events observed in biological systems, thus it is expected to be strongly reduced or even suppressed by DOX treatment. Therefore, the effect of anthracyclines on dividing T-cells urges investigation to improve DOX-mediated antitumor immunity.

This strategy could be promising for complete tumor eradication and might generate a long term immunosurveillance of recurrences and metastases.

From the above description and the above-noted examples, the advantage attained by the uses and methods described and obtained according to the present invention are apparent.

### REFERENCES

Bellini M, Mazzucchelli S, Galbiati E, Sommaruga S, Fiandra L, Truffi M, Rizzuto MA, Colombo M, Tortora P, Corsi F, Prosperi D. J Control Release. 2014 Dec 28;196:184-96. doi: 10.1016/j.jconrel.2014.10.002. Epub 2014 Oct 13.
Mazzucchelli S, Bellini M, Fiandra L, Truffi M, Rizzuto MA, Sorrentino L, Longhi E, Nebuloni M, Prosperi D, Corsi F. Oncotarget. 2017 Jan 31;8(5):8383-8396. doi: 10.18632/oncotarget.14204
Ma, Y., et al., Anticancer chemotherapy-induced intratumoral recruitment and differentiation of antigen-presenting cells. Immunity, 2013. 38(4): p. 729-41.
Reuschenbach, M., M. von Knebel Doeberitz, and N. Wentzensen, A systematic review of humoral immune responses against tumor antigens. Cancer Immunol Immunother, 2009. 58(10): p. 1535-44.
Mattarollo, S.R., et al., Pivotal role of innate and adaptive immunity in anthracycline chemotherapy of established tumors. Cancer Res, 2011. 71(14): p. 4809-20.
Bracci, L., et al., Immune-based mechanisms of cytotoxic chemotherapy: implications for the design of novel and rationale-based combined treatments against cancer. Cell Death Differ, 2014. 21(1): p. 15-25.
Tsung, K. and J.A. Norton, In situ vaccine, immunological memory and cancer cure. Hum Vaccin Immunother, 2016. 12(1): p. 117-9.
Verma, R., et al., Lymphocyte depletion and repopulation after chemotherapy for primary breast cancer. Breast Cancer Res, 2016. 18(1): p. 10.

## Claims

1. Ferritin nanoparticle comprising a chemotherapeutic agent, for use in the preservation of T cells in a cancer patient, wherein said chemotherapeutic agent is a taxane.

2. The use according to claim 1, wherein said T cell preservation is in the treatment of the recurrence of cancer.

3. The use according to anyone of claims 1 or 2, wherein said cancer patient is a patient suffering from breast cancer, bladder, lung, stomach, ovarian cancer; Hodgkin's lymphoma, non-Hodgkin's lymphoma, certain types of leukemia, including acute lymphoblastic leukemia (ALL) and acute myeloid leukemia (AML, ANLL). It is used with other drugs in the treatment of certain types of thyroid cancer and certain types of soft tissue or bone sarcomas, neuroblastoma and Wilms' tumor.

4. The use according to anyone of claims 1 to 3, wherein said cancer patient is suffering from a primary cancer.
